# EUROPÄISCHE PATENTSCHRIFT

(11) **EP 0 611 572 B1**
(45) Veröffentlichungstag und Bekanntmachung des Hinweises auf die Patenterteilung: **07.06.2000**
(21) Anmeldenummer: 94101672.7
(22) Anmeldetag: 04.02.1994
(51) Int. Cl.: A61K 38/09, A61K 47/12

(54) **Herstellungsverfahren für Cetrorelix Lyophilisat**
Process to prepare a cetrorelix lyophilised composition
Procédé de préparation d'une composition lyophilisée de cetrorelix

(30) Priorität: 19.02.1993 DE 4305225
(43) Veröffentlichungstag der Anmeldung: 24.08.1994
(60) Teilanmeldung: 99102340.9
(73) Patentinhaber: ASTA Medica Aktiengesellschaft, 01277 Dresden (DE)
(72) Erfinder: Engel, Jürgen, Prof., D-63755 Alzenau (DE); Sauerbier, Dieter, D-33824 Werther (DE); Wichert, Burkhard, Dr., D-33615 Bielefeld (DE); Reissmann, Thomas, Dr., D-60437 Frankfurt (DE)
(74) Vertreter: Decker, Karl-Ludwig

(56) Entgegenhaltungen:
- EP-A- 0 175 506
- EP-A- 0 268 066
- EP-A- 0 299 402
- WO-A-91/19743
- WO-A-92/08733
- DD-A- 141 996
- FR-A- 2 189 418
- GB-A- 2 125 408
- PROCEEDINGS OF THE NATIONAL ACADEMY OF SCIENCES OF USA., Bd.85, 1988, WASHINGTON US Seiten 1637 - 1641 BAJUSZ S. ET AL 'Highly potent antagonists of luteinizing hormone-releasing hormone free of edematogenic effects'
- JOURNAL OF CANCER RESEARCH AND CLINICAL ONCOLOGY, 1992, 118:44-49

## Beschreibung

Cetrorelix ist ein Dekapeptid mit einer endständigen Säureamidgruppe, das als Acetatsalz eingesetzt wird. Die Synthese und einige pharmakologische Wirkungen sind in der EP-Anmeldung 299 402 beschrieben. Die chemische Struktur von Cetrorelix (SB-75) ist: (Ac-D-NaL(2)¹, D-phe (4Cl)², D-PaL (3)³, D-Cit⁶, D-Ala¹⁰)-LHRH (vergl. J. CANCER RES. CLIN. ONCOL. (1992) 118:44-49).
Der Wirkstoff soll subcutan in einer Dosis von 0,1 bis 20 mg appliziert werden können. Die wässrigen Lösungen des Dekapeptids sind instabil, eine Autoklavierung im Endbehälter ist nicht möglich. Das Dekapeptid neigt unter arzneibuchüblicher Sterilisation zur Zersetzung. Um eine injizierbare Lösung zu erhalten, war daher die Entwicklung eines Lyophilisats erforderlich.
Allerdings ist die Menge des Wirkstoffes in der zu lyophilisierenden Lösung so gering, daß bei niedrigen Wirkstoffkonzentrationen nur ein lockerer Flaum an der Glaswand der Ampulle nach der Trocknung der hilfsstofffreien Lösung resultiert der mit dem zur Sterilisation benutzten Wasserdampfstrom aus dem Vial ausgetragen wird. Es ist also erforderlich, einen Gerüstbildner einzusetzen, der einen stabilen Kuchen bildet. Bei hohen Konzentrationen kann auf diesen Hilfsstoff verzichtet werden. Als Gerüstbildner kommen folgende Hilfsstoffe infrage: Hexite, insbesondere Mannit, Glucit, Sorbit, wie D-Sorbit, Dulcit, Allit, Altrit (zum Beispiel D-und L-Altrit), Idit (zum Beispiel D- und L-Idit), deren optisch aktive Formen (D- beziehungsweise L-Formen) sowie die entsprechenden Racemate. Insbesondere wird Mannit, wie D-Mannit, L-Mannit, DL-Mannit, Sorbit und/oder Dulcit verwendet, und zwar hiervon vorzugsweise D-Mannit. Als Hexit können auch Mischungen der genannten Hexite verwendet werden, zum Beispiel Mischungen von Mannit und Sorbit und/oder Dulcit. Da Dulcit weniger wasserlöslich ist als beispielsweise Mannit, soll der Dulcitgehalt in der wässrigen Lösung beispielsweise 3 Gewichtsprozent nicht überschreiten. Mannit und Sorbit hingegen können beispielsweise in allen Verhältnissen gemischt werden.

Neben dem Hexit können auch noch andere, übliche pharmazeutische Hilfsstoffe zugeführt werden, wie zum Beispiel Aminosäuren, wie beispielsweise Alanin, Glycin, Lysin, Phenylalanin, Asparaginsäure, Glutaminsäure, Leucin, Lactose, Polyvinylpyrrolidon, Glukose, Fructose, Albumin und äquivalente gerüstbildende Stoffe. Weiter können Harnstoff und Natriumchlorid als Gerüstbildner verwendet werden. Die Gesamtmenge an solchen Stoffen in der Lösung, die für die Gefriertrocknung eingesetzt wird, ist beispielsweise 0 - 16,9 Gewichtsteile, beispielsweise 0,1 - 7 Gewichtsteile, bezogen auf 1 Gewichtsteil Cetrorelix. In dem fertigen Lyophilisat kann die Gesamtmenge an solchen Hilfsstoffen bis zu 16,9 Gewichtsteile, bezogen auf einen Gewichtsteil Hexit, betragen. Im einzelnen richtet sich die Menge an solchen Hilfsstoffen nach der vorhandenen Menge Hexit und zwar derart, daß die Gesamtmenge an Hexit und solchen anderen Hilfsstoffen in dem fertigen Lyophilisat maximal nicht mehr als 17 Gewichtsteile beträgt, bezogen auf 1 Gewichtsteil Cetrorelix. Falls in dem Lyophilisat nur 0,1 Gewichtsteile Hexit vorliegen, können also bis zu 16,9 Gewichtsteile an anderen Hilfsstoffen vorliegen; falls beispielsweise 8,5 Gewichtsteile Hexit vorliegen, kann zum Beispiel die Menge an anderen Hilfsstoffen bis zu 8,5 Gewichtsteile, bezogen auf 1 Gewichtsteil Cetrorelix, betragen.

Während der Entwicklungsarbeiten zu dem Lyophilisat mußte jedoch festgestellt werden, daß sich der Wirkstoff bei der Verarbeitung sehr unterschiedlich und nicht vorhersehbar verhält. Die ersten Ansätze führten zu guten Resultaten, bald jedoch stellte sich heraus, daß bei der Sterilfiltration Schwierigkeiten auftreten und Fehlchargen resultierten.

Aus der Literatur beispielsweise aus Powell. M. F.: Pharmaceutical Resarch, 1258-1263 (8) 991; Dathe, M.: Int. J. Peptide Protein Res. 344-349 (36) 1990; Szejtli, J.: Pharmaceutical Technology International 16-22, 1991 ist bekannt, daß Oligopeptide, besonders solche mit endständiger Säureamidfunktion, zur Gelbildung neigen. Bei der Sterilfiltration ist dies an der Filtrationsgeschwindigkeit erkennbar, oft erkennt man sogar schon organoleptisch die erhöhte Viskosität derartiger Lösungen. Auf dem Sterilfilter bleibt eine gallertartige Schicht zurück. Damit ist es nicht mehr möglich, ein Arzneimittel mit einem genau definierten Gehalt an Wirkstoff herzustellen.

In der Tabelle 1 sind verschiedene Ergebnisse der ersten 11 Ansätze aufgelistet.

Die Wirkstoffgehalte schwanken zwischen 100 % und 36 %.

**Tabelle 1**

| Cetrorelix-Acetat | | |
|---|---|---|
| Charge | Dosierung | Wirkstoffgehalt |
| 1 | 100 µg | 100 |
| 2 | 500 µg | 100 |
| 3 | 500 µg | 90 |
| 4 | 500 µg | 36 |
| 5 | 500 µg | 100 |
| 6 | 500 µg | 85 |
| 7 | 1 mg | 80 |
| 8 | 1 mg | 100 |
| 9 | 2 mg | 100 |
| 10 | 2 mg | 80 |
| 11 | 2 mg | 100 |

Um diese Gelbbildung zu vermeiden, werden in der Literatur folgende Zusatzstoffe aufgeführt, die versuchsweise eingesetzt wurden.

Es kommen organische Lösungsmittel infrage, beispielsweise Acetonitril, n-Butanol, tertiäres Butanol, Ethanol, Isopropanol, Octanol und Benzylalkohol. Weiter können Salze und Pufferlösungen verwendet werden, wie zum Beispiel Acetatpuffer, Citratpuffer, Natriumchlorid, Natriumphosphat. Natrium EDTA, Natriumbicarbonat, Phosphatpuffer, Guanidinacetat, Harnstoff.
Weitere Anwendung können Polymere finden, wie zum Beispiel Gelatine, Polyethylenglykol 600, Hydroxyethylstärke, Polyvinylpyrrolidon, Polyvinylalkohol. Auch der Zusatz von Aminosäuren, beispielsweise Alanin, Glycin, Lysin, Phenylalanin, Asparaginsäure, Glutaminsäure und Leucin wurde schon beschrieben. An Säuren wurde eingesetzt Citronensäure, Caprylsäure, Octansäure, Salzsäure, Schwefelsäure und Essigsäure. An physiologisch unbedenklichen Tensiden stehen Benzalkoniumchlorid, Cetylalkohol, Gallensäuren, Lecithine, Polysorbate, Spans^{(R)} und Pluronics^{(R)} zur Verfügung.
Auch Kohlehydrate und Cyclodextrine wie zum Beispiel Glukose, Lactose, Mannitol, Saccharose, alpha-, beta- und gamma Cyclodextrine, Hydroxypropyl-alpha-und beta-Cyclodextrine, Hydroxyethyl Cyclodextrine und Methyl-Cyclodextrine wurden schon eingesetzt. Diese Hilfsstoffe wurden als Filtrationshilfsmittel zur Vermeidung der Gelbildung erprobt.

Eine befriedigende Lösung des Problems war jedoch nicht zu erkennen. Lediglich ein Ansäuern mit Essigsäure zeigte Teilerfolge. Aber auch hier mußten immer wieder höhere Filtrationsverluste hingenommen werden.
Überraschenderweise stellte sich nun heraus, daß man Cetrorelix in 30 %iger Volumen/Volumen Essigsäure gut lösen kann. Diese Lösung wird anschließend auf eine Endkonzentration von 3 % Cetrorelix mit Wasser für Injektionszwecke aufgefüllt und Mannit hinzugegeben. Obwohl in der Literatur beschrieben wird, daß in saurem Medium die endständige Amidgruppe leicht hydrolysiert, konnte dies beim Cetrorelix nicht festgestellt werden. Lösungen, die nach dieser Methode hergestellt werden, machten bei der Filtration keinerlei Schwierigkeiten. Es wurden immer die korrekten Wirkstoffgehalte gefunden.
Die Filtrationsgeschwindigkeit erreicht Werte, die befriedigende Produktionsabläufe sicherstellen. Ein allgemeiner Prozeß zur sterilen Lyophilisation wird in Sucker, Fuchs und Speiser (Herausgeber) 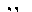 Pharmazeutische Technologie" 2. Auflage 1991, Thieme-Verlag, Stuttgart-New York, auf den Seiten 557-559 beschrieben, Eine weitere Beschreibung des angewandten Lyophilisierungsprozesses findet sich in der DE-OS 37 35 614.

Es wurde nun ein Verfahren zur Herstellung eines sterilen Cetrorelixacetat-Lyophilisates gefunden, was sich dadurch auszeichnet, daß man Cetrorelixacetat in wässriger Essigsäure im Verhältnis 1:100 - 10000 Gew/Gew löst, die Lösung mit Wasser für Injektionszwecke verdünnt, ggf einen Gerüststoff zusetzt, sterilfiltriert, in Injektionsflaschen abfüllt und lyophilisiert. Damit wurden die oben genannten Schwierigkeiten behoben.

Das erfindungsgemäß hergestellte Lyophilisat findet seine Anwendung in der Therapie der weiblichen Sterilität und bei der Gonadenprotektion von männlichen Patienten.

Das Verfahren wird anhand des nachfolgenden Ausführungsbeispiels näher beschrieben ohne es jedoch damit einzuschränken.

Beispiel für das Herstellverfahren:

In ein geeignetes Glasgefäß werden ca. 1.5 Liter Wasser für Injektionszwecke vorgelegt. In ein weiteres Glasgefäß werden 210 g Wasser für Injektionszwecke vorgelegt und 91,17 g Essigsäure zugefügt. Die berechnete Menge Cetrorelixacetat (1,62 - 1,695 g, je nach Gehalt der eingesetzten Charge) wird in der hergestellten 30 %igen Essigsäure unter Rühren gelöst. Die Lösung wird in das Glasgefäß mit 1,5 Liter Wasser für Injektionszwecke überführt, 82,2 g Mannitol zugegeben, gelöst und mit Wasser für Injektionszwecke auf 3039 g aufgefüllt.

Inprozeßkontrollen:

| | |
|---|---|
| pH-Wert: | 2,5 - 3,0 |
| Dichte: | 1,009 - 1,017 g/cm³ bei 20 °C |
| Brechungsindex: | 1,227 - 1,340 bei 440 nm und 20 °C |

Die Sterilisationen der Lösung erfolgt durch Filtration über ein geeignetes Membranfilter (Porenweite 0,2 µm) unter aseptischen Bedingungen. 100 ml Vorlauf sind zu verwerfen. Die Filter sind mit gespannten Wasserdampf zu sterilisieren. Cetrorelix Gefriertrocknungslösung wird vor Rekontamination geschützt aufbewahrt. Die Lösung wird unverzüglich in Injektionsflaschen DIN 2R farblos, hydrolytische Klasse I unter aseptischen Bedingungen dosiert und mit sterilen Gefriertrocknungsstopfen versehen. Die Sollfüllmenge beträgt 2,0 ml = 2,026 g.

Die 2 ml Injektionsflaschen wurden auf einer Injektionsflaschenwaschmaschine gespült und mit Heißluft getrocknet und sterilisiert. Die gereinigten Gefriertrocknungsstopfen wurden autoklaviert. Die vorverschlossenen Injektionsflaschen wurden in eine Gefriertrocknungsanlage überführt und bei einer Plattentemperatur von -40 °C auf +20 °C steigend Anschließend wird die Anlage mit sterilem Stickstoff geflutet, die Flaschen in der Anlage verschlossen, und die Stopfen mit Bördelkappen gesichert.

Die Injektionsflaschen werden visuell auf Verschlußfehler und äußere Fehler kontrolliert. Fehlerhafte Injektionsflaschen werden aussortiert und vernichtet.

Cetrorelix Lyophilisat 1 mg ist ein weißer, fester Gefriertrocknungskuchen in einer farblosen 2 ml Injektionsflasche, die mit grauen Gefriertrocknungsstopfen und gelber Flipp-off-Bördelkappe verschlossen ist.

## Patentansprüche

1. Verfahren zur Herstellung eines sterilen Cetrorelixacetat-Lyophilisates, woein Cetrorelix für (Ac-D-Nal(2)', D-phe(4Cl)², D-PaL(3)³, D-Cit⁶, D-Ala¹⁰)-LHRH steht, dadurch gekennzeichnet, daß man Cetrorelixacetat in wässriger Essigsäure im Verhältnis 1:100 - 10000 Gew/Gew löst, die Lösung mit Wasser für Injektionszwecke verdünnt, ggf. einen Gerüststoff zusetzt, sterilfiltriert, in Injektionsflaschen abfüllt und lyophilisiert.

## Claims

1. Process for the preparation of a sterile cetrorelix acetate lyophilizate, in which cetrorelix is (Ac-D-Nal(2)¹, D-phe(4Cl)², D-PaL(3)³, D-Cit⁶, D-Ala¹⁰)-LHRH, characterized in that cetrorelix acetate is dissolved in aqueous acetic acid in the ratio 1:100-10,000 wt/wt, the solution is diluted with water for injection, if appropriate a builder is added, and the solution is sterile-filtered, filled into injection vials and lyophilized.

## Revendications

1. Procédé de préparation d'un lyophilisat d'acétate de Cétrorelix stérile dans lequel Cétrorelix représente Ac-D-Nal(2)¹, D-p
he(4Cl)², D-Pal(3)³, D-Cit⁶, D-Ala¹⁰)-LHRH,
caractérisé en ce qu'
on dissout l'acétate de Cétrorelix dans de l'acide acétique aqueux dans un rapport allant de 1:100 à 10.000, poids/poids, on dilue la solution avec de l'eau pour injection, on ajoute éventuellement une substance de structuration, on filtre d'une manière stérilisante, on remplit en flacons pour injection et on lyophilise.
